# FASCICULE DE BREVET EUROPEEN

(11) **EP 1 872 704 B1**
(45) Date de publication et mention de la délivrance du brevet: **17.02.2010**
(21) Numéro de dépôt: 07111463.1
(22) Date de dépôt: 29.06.2007
(51) Int. Cl.: A47L 15/48

(54) **Machine à laver et à sécher la vaisselle équipé d'un dispositif de traitement des odeurs**
Maschine zum Spülen und Trocknen von Geschirr, die mit einer Vorrichtung zur Geruchsbehandlung ausgestattet ist
Dishwasher and dryer equipped with a device for treating smells

(30) Priorité: 30.06.2006 FR 0606048
(43) Date de publication de la demande: 02.01.2008
(73) Titulaire: FagorBrandt SAS, 92500 Rueil Malmaison Cedex (FR)
(72) Inventeur: Tissot, Carène, 69007 Lyon (FR); Bretaud, Jacques, 85000 Mouilleron le Captif (FR); Vanhulle, Faustine, 69007 Lyon (FR); Pham Van Chu, Michel, 69200 Venissieux (FR); Geoffroy, Philippe, 45370 Cléry Saint André (FR)

(56) Documents cités:
- DE-A1- 3 921 177
- JP-A- 3 126 423
- JP-A- 3 234 234
- JP-A- 7 079 906
- JP-A- 11 137 498
- JP-A- 2005 118 209

## Description

La présente invention concerne une machine à laver et à sécher la vaisselle équipée d'un dispositif de traitement des odeurs, et plus particulièrement par photocatalyse.

Elle concerne de manière générale les machines à laver et à sécher la vaisselle domestiques comprenant une cuve de lavage reliée à un circuit de ventilation pour le séchage de la vaisselle.

Traditionnellement, les machines à laver la vaisselle peuvent permettre de masquer les odeurs en accueillant une cartouche de parfum se diffusant dans la cuve de lavage. Cet accessoire est acheté par l'utilisateur et mis en place à l'intérieur de la cuve de lavage en le suspendant par exemple à un panier de chargement de la vaisselle.

Ces cartouches de parfum sont à recharger régulièrement et ont un coût relativement élevé.

Cependant, ces cartouches de parfum ne permettent pas de détruire les mauvaises odeurs mais seulement de les masquer avec une efficacité faible. En outre, les utilisateurs considèrent les odeurs dégagées par les cartouches de parfum comme « chimiques » et relativement désagréables à sentir.

On connaît également des produits de lavage et / ou de rinçage parfumant pour les machines à laver la vaisselle. Ces produits ont l'inconvénient de se déposer sur la vaisselle en même temps que sur les parois de la machine à laver la vaisselle. Les odeurs diffusées sur la vaisselle peuvent indisposer certains utilisateurs.

On connaît également le document JP 11 137498 A qui décrit une machine à laver la vaisselle équipée d'un catalyseur permettant la désodorisation de l'air provenant de la cuve de ladite machine. Ce catalyseur est placé dans un canal d'évacuation de l'air vers l'extérieur.

La présente invention a pour but de résoudre les inconvénients précités et de proposer une machine à laver et à sécher la vaisselle permettant de détruire les mauvaises odeurs. La désodorisation de la machine à laver et à sécher la vaisselle consiste à détruire les molécules organiques odorantes en les craquant et en les minéralisant. Ce principe de désodorisation est basé sur la photocatalyse. Ainsi, les mauvaises odeurs de vaisselle sale stockée dans une cuve de lavage sont supprimées entre deux cycles de lavage de la vaisselle.

A cet effet, la présente invention vise, selon un premier aspect, une machine à laver et à sécher la vaisselle comprenant une cuve de lavage ayant une paroi de fond, des parois latérales et une paroi supérieure formant une cavité pour le lavage de la vaisselle, dont la face frontale de la cavité est fermée par une porte.

Selon l'invention, la machine à laver et à sécher la vaisselle comprend un dispositif de traitement des odeurs, ledit dispositif de traitement des odeurs comprenant au moins un catalyseur associé à une couche de charbon actif, et ledit au moins un catalyseur étant irradié par au moins une source de rayons ultraviolets, et en ce que ledit dispositif de traitement des odeurs est placé à l'intérieur d'un conduit de ventilation du circuit de séchage de ladite machine.

Ainsi, les molécules organiques odorantes entrent en contact avec une surface recouverte d'au moins un catalyseur. Ladite surface recouverte d'au moins un catalyseur est irradiée par au moins une source de rayons ultraviolets, ladite au moins une source de rayons ultraviolets pouvant être au moins une diode électroluminescente émettant des rayons ultraviolets de type A. Les molécules organiques odorantes sont alors minéralisées.

Le dispositif de traitement des odeurs est efficace tout au long de la durée de vie de la machine à laver et à sécher la vaisselle sans avoir à changer un élément ou à recharger un produit.

Le dispositif de traitement des odeurs placé à l'intérieur de la machine à laver et à sécher la vaisselle permet de capter et de traiter rapidement et efficacement les mauvaises odeurs enfermées dans ladite machine.

Le dispositif de traitement des odeurs peut fonctionner périodiquement entre les cycles de lavage de la machine à laver et à sécher la vaisselle pendant une durée prédéterminée ou suivant une durée calculée par un moyen de commande de ladite machine, ou encore suivant une commande ordonnée par un utilisateur.

Le dispositif de traitement des odeurs est placé à l'intérieur d'un conduit de ventilation du circuit de séchage de ladite machine.

Ainsi, les odeurs contenues dans la machine à laver et à sécher la vaisselle sont aspirées et circulent à travers un conduit de ventilation. La circulation d'air à l'intérieur de la machine à laver et à sécher la vaisselle est réalisée par au moins un ventilateur. Ladite circulation d'air de la machine à laver et à sécher la vaisselle est dite forcée.

Le dispositif de traitement des odeurs est placé à l'intérieur d'un conduit de ventilation déjà existant sur les machines à laver et à sécher la vaisselle classiques utilisant un mode de séchage par convection forcée. De cette manière, le dispositif de traitement des odeurs peut être intégré à une machine classique sans modifications structurelles engendrant un coût supplémentaire.

Le circuit de ventilation de la machine à laver et à sécher la vaisselle peut être fermé ou encore légèrement ouvert pour permettre une entrée d'air extérieur.

De manière nullement limitative, l'entrée d'air extérieur peut permettre d'introduire de l'air extérieur à la machine à laver et à sécher la vaisselle de l'ordre de 10 à 15% du volume d'air du circuit de ventilation. Le renouvellement d'air à l'intérieur de la machine à laver et à sécher la vaisselle par de l'air extérieur s'effectue entre au moins une ouverture d'entrée d'air et au moins une ouverture de sortie d'air reliées au circuit de ventilation de ladite machine.

Le dispositif de traitement des odeurs est inséré dans un conduit de ventilation du circuit de séchage de la machine à laver et à sécher la vaisselle sans créer de perturbations aérauliques. L'implantation du dispositif de traitement des odeurs est inséré dans un conduit de ventilation du circuit de séchage de la machine à laver et à sécher la vaisselle ne génère pas de pertes de charges supplémentaires.

Entre deux cycles de lavage, le dispositif de traitement des odeurs peut fonctionner en simultané avec ledit au moins un ventilateur de la machine à laver et à sécher la vaisselle. Ledit au moins un ventilateur assurant la circulation d'air interne traversant la cuve de lavage peut fonctionner en continu ou en discontinu lors d'un cycle de désodorisation de la machine à laver et à sécher la vaisselle.

Le dispositif de traitement des odeurs doit fonctionner régulièrement pour empêcher que la vaisselle sale rangée dans la cuve de lavage de la machine à laver et à sécher la vaisselle ne dégage de mauvaises odeurs.

Le dispositif de traitement des odeurs peut fonctionner aux périodes précédant les repas, notamment aux périodes précédant le remplissage de la machine à laver et à sécher la vaisselle. Ces périodes nécessitent une destruction efficace des odeurs avant d'ouvrir la porte de la machine à laver et à sécher la vaisselle. La durée de fonctionnement du dispositif de traitement des odeurs peut être de l'ordre de 30 minutes à deux heures en continu, et préférentiellement de l'ordre d'une heure et demie.

Le dispositif de traitement des odeurs comprend au moins un catalyseur associé à une couche de charbon actif.

Ainsi, le support du dispositif de traitement des odeurs comprend au moins une couche d'au moins un catalyseur tel que le dioxyde de titane et au moins une couche de charbon actif. La fonction de ladite au moins une couche de charbon actif est de servir de tampon et d'adsorber les pics importants d'odeurs. Le charbon actif est simultanément régénéré par ladite au moins une couche de catalyseur sous l'irradiation de ladite au moins une source de rayons ultraviolets.

Une partie des molécules organiques odorantes sont piégées dans une couche de charbon actif jouant le rôle de tampon et une autre partie des molécules organiques odorantes entrent en contact avec une surface dudit au moins un catalyseur irradiée par ladite au moins une source de rayons ultraviolets pour être minéralisées. Lesdites molécules organiques odorantes piégées dans la couche de charbon actif se déplacent vers la couche dudit au moins un catalyseur dès que l'autre partie des molécules organiques odorantes sont détruites.

Ladite au moins une couche de charbon actif doit être préférentiellement sèche pour adsorber les molécules organiques odorantes.

Dans le cas où le charbon actif est mouillé, ce dernier peut être séché par ledit au moins un ventilateur du circuit de séchage de la machine à laver et à sécher la vaisselle.

Selon une autre caractéristique préférée de l'invention, ledit au moins un catalyseur est du dioxyde de titane.

La quantité dudit au moins un catalyseur, pouvant être du dioxyde de titane, peut être comprise dans une plage s'étendant entre 15 et 20 grammes. Bien entendu, cette quantité dudit au moins un catalyseur n'est nullement limitative.

Ledit au moins un catalyseur permet le fonctionnement du dispositif de traitement des odeurs même en étant mouillé.

Préférentiellement, ledit au moins un catalyseur est déposé sur un support non tissé de forme plane.

De cette manière, le support rentre aisément dans un conduit de ventilation de la machine à laver et à sécher la vaisselle sans modification de ladite machine et en ne créant pas de pertes de charges supplémentaires.

Ce support permet d'utiliser le dispositif de traitement des odeurs même en étant mouillé régulièrement et en ne se dégradant pas au cours de la durée de vie de la machine à laver et à sécher la vaisselle.

Selon une autre caractéristique préférée de l'invention, un flux d'air à l'intérieur du conduit de ventilation est tangentiel au support dudit au moins un catalyseur.

Ainsi, le flux d'air à l'intérieur du conduit de ventilation lèche au moins une surface dudit au moins un catalyseur pouvant être irradiée au même instant par ladite au moins une source de rayons ultraviolets. Dans ce mode de réalisation de l'invention, le flux d'air du conduit de ventilation ne traverse pas le support dudit au moins un catalyseur.

Préférentiellement, le rayonnement de ladite au moins une source de rayons ultraviolets sur ledit au moins un catalyseur s'effectue au travers d'une paroi transparente aux rayonnements ultraviolets du conduit de ventilation.

Ainsi, le positionnement de ladite au moins une source de rayons ultraviolets permet d'obtenir un rayonnement à travers un matériau transparent aux rayons ultraviolets. Ladite au moins une source de rayons ultraviolets est isolée du circuit de ventilation et de l'humidité. Le matériau de la paroi transparente aux rayonnements ultraviolets du conduit de ventilation peut être un polypropylène transparent laissant passer au moins 60% des rayons ultraviolets de type A pour une épaisseur de l'ordre de 1,5mm.

En pratique, ladite au moins une source de rayons ultraviolets est placée à l'extérieur du conduit de ventilation.

Dans un autre mode de réalisation de l'invention, ladite au moins une source de rayons ultraviolets peut être positionnée de manière à avoir un rayonnement direct sur le support du dispositif de traitement des odeurs comprenant au moins un catalyseur. Dans ce cas, ladite au moins une source de rayons ultraviolets est en contact direct avec l'humidité résiduelle du circuit de ventilation de la machine à laver et à sécher la vaisselle. D'où la nécessite de protéger électriquement ladite au moins une source de rayons ultraviolets et les moyens de commande de cette dernière par exemple par une couche de résine. Les moyens de commande de ladite au moins une source de rayons ultraviolets peuvent être au moins une carte électronique, ladite carte électronique peut comprendre au moins un microcontrôleur.

Selon une autre caractéristique préférée de l'invention, le support dudit au moins un catalyseur est placé à distance d'une paroi inférieure et d'une paroi supérieure du conduit de ventilation.

Ainsi, le support comprenant ledit au moins un catalyseur est positionné au travers du flux d'air du conduit de ventilation. De cette manière, les molécules organiques odorantes circulent sur les deux faces dudit support non tissé et de forme plane. En outre, la distance séparant ledit support de ladite au moins une source de rayons ultraviolets est réduite et permet une irradiation dudit au moins un catalyseur plus puissante.

Le dispositif de traitement des odeurs peut être activé par une touche pour un fonctionnement manuel ou automatique. Cette touche permet également de débrayer le fonctionnement du dispositif de traitement des odeurs.

La présente invention concerne également, selon un second aspect, un procédé de traitement des odeurs dans une machine à laver et à sécher la vaisselle telle que décrite précédemment.

Ladite machine à laver et à sécher la vaisselle comprend une cuve de lavage ayant une paroi de fond, des parois latérales et une paroi inférieure formant une cavité pour le lavage de la vaisselle, dont la face frontale de la cavité est fermée par une porte.

Ladite machine à laver et à sécher la vaisselle comprend également un dispositif de traitement des odeurs, ledit dispositif de traitement des odeurs comprenant au moins un catalyseur, et ledit au moins un catalyseur étant irradié par au moins une source de rayons ultraviolets.

Le dispositif de traitement des odeurs fonctionne périodiquement.

Ainsi, le dispositif de traitement des odeurs fonctionne par cadence pour réduire les coûts liés à la durée de vie des composants et éviter tout remplacement d'un élément de la machine.

La machine à laver et à sécher la vaisselle est conçue en prédéterminant une durée de fonctionnement dudit au moins un ventilateur pour faire circuler les odeurs dans la machine, une durée d'allumage de ladite au moins une source de rayons ultraviolets pour supprimer les odeurs par traitement photocatalytique, et une durée d'arrêt du dispositif de traitement des odeurs et dudit au moins un ventilateur de la machine à laver et à sécher la vaisselle.

Le temps d'arrêt du dispositif de ventilation et dudit au moins un ventilateur doit être d'une durée inférieure à la durée de propagation des odeurs de vaisselle sale dans la machine à laver et à sécher la vaisselle. En effet, les salissures présentes sur la vaisselle renouvellent les mauvaises odeurs dans la cuve de lavage rapidement. La demanderesse a pu constater que la machine à laver et à sécher la vaisselle est de nouveau polluée au bout de quelques heures.

Préférentiellement, le dispositif de traitement des odeurs se déclenche après chaque ouverture de la porte de la machine.

Chaque ouverture de porte de la machine à laver et à sécher la vaisselle correspond généralement à une introduction de vaisselle sale supplémentaire.

La durée de traitement des odeurs est comprise dans une plage s'étendant entre 4 minutes et 20 minutes par heure.

Le dispositif de traitement des odeurs se déclenche de manière cyclique en dehors des cycles de lavage de ladite machine.

Le dispositif de traitement des odeurs se met en veille après une période prédéterminée de non ouverture de la porte de ladite machine.

Dans ce cas, l'utilisateur est considéré comme absent pour une période longue et à la prochaine ouverture de porte le dispositif de traitement des odeurs se réveille comme décrit précédemment.

La machine à laver et à sécher la vaisselle comprend au moins une ouverture d'entrée d'air dans une paroi de la cuve de lavage permettant l'aspiration des odeurs par au moins un ventilateur, lesdites odeurs circulant dans un conduit de ventilation et traversant le dispositif de traitement des odeurs par photocatalyse, puis l'air traité par ledit dispositif de traitement des odeurs par photocatalyse étant évacué au travers d'au moins une ouverture de sortie dans une paroi de la cuve de lavage.

Ledit au moins un ventilateur et ladite au moins une source de rayons ultraviolets fonctionnent simultanément pour le traitement des odeurs.

Ladite au moins une source de rayons ultraviolets fonctionne pendant une durée supplémentaire à une durée de traitement des odeurs pour régénérer une couche de charbon actif déposée sur un support comprenant ledit au moins un catalyseur.

La régénération dudit charbon actif est réalisée sans la mise en fonctionnement dudit au moins un ventilateur.

La durée de fonctionnement du dispositif de traitement des odeurs pour détruire les odeurs et la durée de fonctionnement supplémentaire pour régénérer ladite couche de charbon actif peuvent être réalisées en continu. Ainsi, ladite au moins une source de rayons ultraviolets est allumée en continu.

Ladite au moins une source de rayons ultraviolets fonctionne pendant une durée supplémentaire pour régénérer une couche de charbon actif déposée sur un support comprenant ledit au moins un catalyseur comprise dans une plage s'étendant entre 4 minutes et 10 minutes, et préférentiellement de l'ordre de 8 minutes.

D'autres particularités et avantages de l'invention apparaîtront encore dans la description ci-après.

Aux dessins annexés, donnés à titre d'exemples non limitatifs :
- la figure 1 est une vue en perspective d'une machine à laver et à sécher la vaisselle conforme à l'invention ;
- la figure 2 est une vue éclatée d'un dispositif de traitement des odeurs et d'un dessus de cuve de lavage d'une machine à laver et à sécher la vaisselle conforme à l'invention ;
- la figure 3 est une vue de dessus d'un dispositif de traitement des odeurs et d'un dessus de cuve de lavage d'une machine à laver et à sécher la vaisselle conforme à l'invention ;
- la figure 4 est une vue en coupe d'un conduit de ventilation comprenant un dispositif de traitement des odeurs d'une machine à laver la vaisselle conforme à un premier mode de réalisation de l'invention ;
- la figure 5 est une vue en coupe d'un conduit de ventilation comprenant un dispositif de traitement des odeurs d'une machine à laver la vaisselle conforme à un second mode de réalisation de l'invention ;
- la figure 6 est une vue en coupe d'un conduit de ventilation comprenant un dispositif de traitement des odeurs d'une machine à laver la vaisselle conforme à un troisième mode de réalisation de l'invention ;
- la figure 7 est une vue en coupe d'un conduit de ventilation comprenant un dispositif de traitement des odeurs d'une machine à laver la vaisselle conforme à un quatrième mode de réalisation de l'invention.

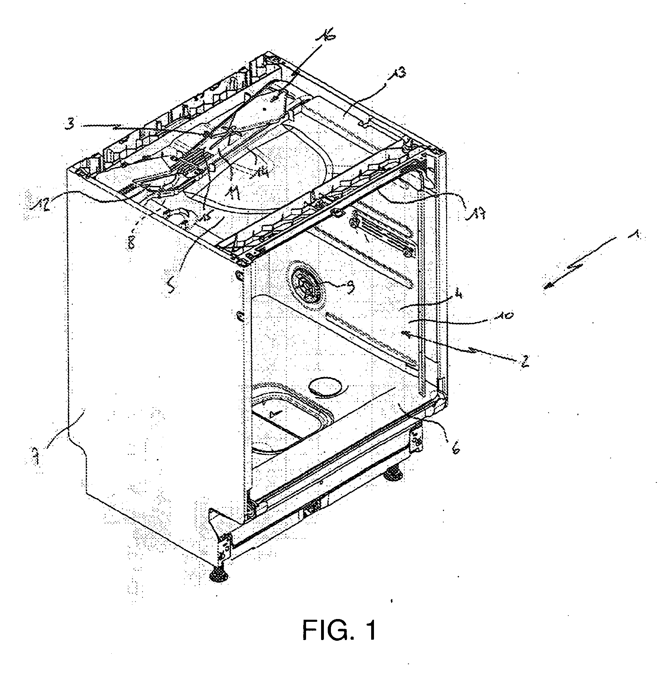

On va décrire tout d'abord, en référence à la figure 1, une machine à laver et à sécher la vaisselle conforme à l'invention.

On a représenté pour décrire l'invention une cuve de lavage 2 d'une machine à laver et sécher la vaisselle 1 conforme à l'invention, montée en communication sur un circuit d'aspiration de l'air de séchage 3 représenté uniquement de manière schématique.

Bien entendu, l'ensemble est décrit dans un boîtier de machine qui n'a pas besoin d'être décrit ici.

En outre, l'ensemble des autres composants nécessaires au fonctionnement d'un lave-vaisselle, tels que, par exemple, les moyens de commande, d'alimentation électrique et en eau, ne sont pas représentés ou décrits dès lors qu'ils peuvent être identiques à ceux bien connu de l'homme du métier dans ce domaine.

Cette machine à laver et sécher la vaisselle 1 comprend tout d'abord une cuve de lavage 2, de forme sensiblement parallélépipédique. Cette cuve de lavage 2 comporte ainsi une enveloppe 4 en forme de U, un plafond 5, une paroi de fond 6 et une porte (non représentée) permettant d'obturer cette cuve de lavage 2. Les parois 10 de l'enveloppe 4 sont adaptées à supporter au moins un panier (non représenté) destiné à recevoir de la vaisselle. De façon connue, les paniers présentent la forme générale d'un parallélépipède rectangle et sont constitués de fils entrecroisés et soudés formant ainsi un fond et une bordure à quatre angles pratiquement droits.

Cette cuve de lavage 2 est montée dans un boîtier 7, représenté partiellement, également de forme parallélépipédique.

De manière classique dans une telle machine à laver et sécher la vaisselle 1, une ouverture d'entrée d'air 8 est ménagée dans le plafond 5 de la cuve de lavage 2 et une ouverture de sortie d'air 9 est ménagée dans une paroi latérale 10 de la cuve de lavage 2, de telle sorte que l'air utilisé lors de la phase de séchage de la vaisselle peut entrer et une fois chargé en humidité être évacué de la cuve de lavage 2. Ces deux ouvertures 8 et 9 sont reliées à un circuit d'aspiration de l'air de séchage 3. Ce circuit d'aspiration de l'air de séchage 3 comporte un conduit de ventilation 11 monté à l'extérieur de la cuve de lavage 2.

Afin d'assurer la circulation de l'air de séchage dans ce conduit de ventilation 11, un ventilateur 12 est monté dans le conduit de ventilation 11 en un endroit approprié.

En outre, un échangeur air - eau 13 est relié au conduit de ventilation 11 à proximité de l'ouverture de sortie d'air de séchage 9. Cet échangeur air - eau 13 peut être de manière connue un condenseur à eau où l'air chargé en humidité entre en contact avec la paroi refroidie par l'eau et une partie de l'humidité est condensée.

Ce mode réalisation permet l'adaptation de plusieurs variantes où le circuit de traitement de l'air 3 peut être fermé, ouvert ou semi-ouvert. Le circuit de traitement de l'air 3 est considéré comme ouvert dès qu'une ouverture d'introduction d'air de séchage apporte de l'air de l'extérieur de manière à renouveler l'air contenu dans la cuve de lavage 2 de la machine 1. Un circuit de traitement de l'air 3 semi-ouvert est un circuit où l'air de l'enceinte de lavage 2 est mélangé avec un apport d'air de l'extérieur, le mélange d'air extérieur et d'air renfermé dans la cuve de lavage 2 est en recirculation au travers du circuit de ventilation 3 de la machine 1.

Enfin, des moyens de chauffage (non représentés) sont généralement placés dans le conduit de ventilation 11 à proximité d'une ouverture d'entrée d'air de séchage 8 dans la cuve de lavage 8 afin d'élever la température de l'air de séchage destiné à venir en contact avec la vaisselle à sécher.

Ces moyens de traitement de l'air de séchage dans le conduit de ventilation 11, constitués d'un échangeur air - eau 13 et un moyen de chauffage, sont seulement indiqués à titre non limitatif dans ce mode de réalisation. Tout autre dispositif de séchage, par condensation ou évaporation, pourrait également être mis en oeuvre dans ce type de machine à laver et sécher la vaisselle 1.

Une machine à laver et sécher la vaisselle 1 comporte aussi un circuit de traitement de l'eau de lavage et rinçage de la vaisselle. Ce circuit de traitement de l'eau de lavage et rinçage n'est pas représenté sur ces figures afin de les rendre plus lisibles. Ce circuit de traitement de l'eau de lavage et rinçage comporte une gaine montée à l'extérieur de la cuve de lavage et mise en communication avec l'intérieur de la cuve de lavage au niveau de la paroi inférieure et de la paroi supérieure de la cuve de lavage.

Une motopompe (non représentée) montée sous la paroi inférieure de la cuve de lavage permet de faire circuler l'eau dans le circuit de traitement de l'eau de lavage et rinçage.

La cuve de lavage 2 peut être produite au moins en partie dans un matériau non ferreux tel que de l'inox amagnétique.

Ladite cuve de lavage 2 peut comprendre un dessus de cuve 5 en matière plastique connecté auxdites parois latérales 10, et lesdites paroi de fond 6 et parois latérales 10 sont en métal et ledit dessus de cuve 5 comprend une partie du circuit d'air de séchage 3.

Ce dessus de cuve 5 étant en plastique et en une seule pièce monobloc peut, par exemple, être réalisé par un procédé de moulage par injection.

Avec un tel procédé de fabrication, le dessus de cuve 5 peut intégrer de nombreux moyens de fixation.

Ici et de manière nullement limitative, la partie du circuit d'air de séchage 3 du dessus de cuve 5 est connecté à un moyen externe de condensation de l'air de séchage 13.

Ici et de manière nullement limitative, la partie du circuit d'air de séchage 3 du dessus de cuve 5 comprend au moins une partie inférieure 14 de conduit d'extraction d'air de séchage 11, une partie inférieure de volute d'un ventilateur 12 et des moyens de fixation de la partie inférieure du conduit d'extraction d'air de séchage 14 avec une partie supérieure du conduit d'extraction d'air de séchage 15.

En outre, la partie du circuit d'air de séchage 11 du dessus de cuve 5 peut comprendre des moyens d'étanchéité.

Ce dessus de cuve 5 permet notamment d'intégrer de nombreuses fonctions, tels que par exemple des moyens de fixation des câbles électriques et des canalisations d'eau, des parois du circuit d'air de séchage, dans le but de réduire le nombre de pièces constituant la machine à laver et à sécher la vaisselle, tel qu'illustré à la figure 1.

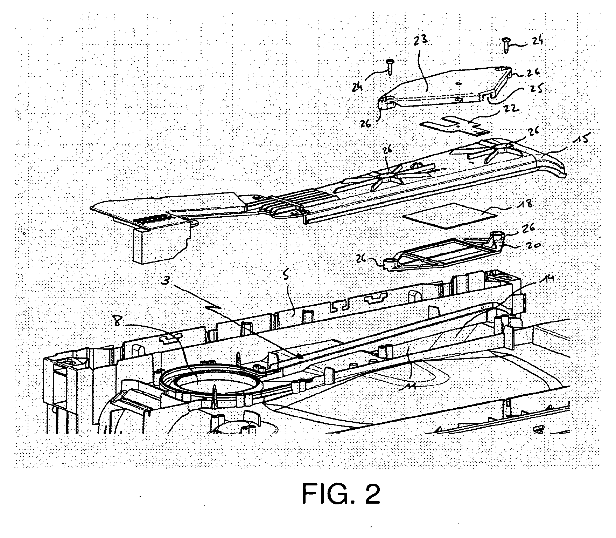

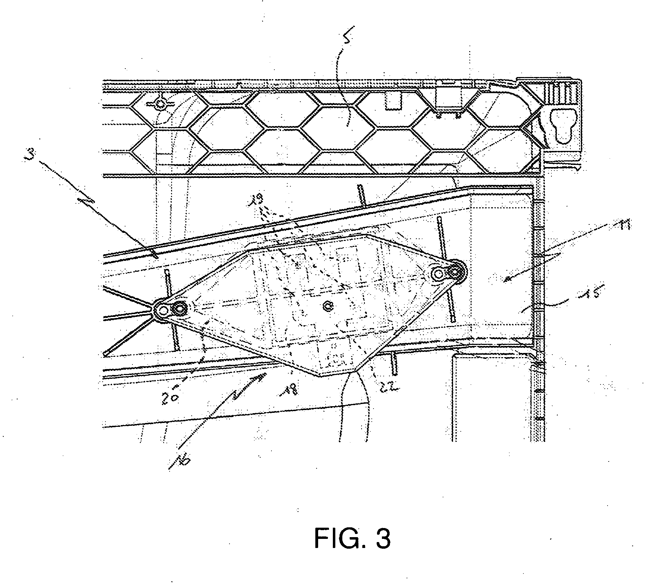

On va décrire à présent, en référence aux figures 2 à 6, le dispositif de traitement des odeurs 16 conforme à l'invention.

Une machine à laver et à sécher la vaisselle 1 comprend une cuve de lavage 2 ayant une paroi de fond 6, des parois latérales 10 et une paroi supérieure 5 formant une cavité pour le lavage de la vaisselle, dont la face frontale 17 de la cavité 2 est fermée par une porte.

La machine à laver et à sécher la vaisselle 1 comprend un dispositif de traitement des odeurs 16, ledit dispositif de traitement des odeurs 16 comprenant au moins un catalyseur 18, et ledit au moins un catalyseur 18 étant irradié par au moins une source de rayons ultraviolets 19.

Ainsi, les molécules organiques odorantes entrent en contact avec une surface recouverte d'au moins un catalyseur 18. Ladite surface recouverte d'au moins un catalyseur 18 est irradiée par au moins une source de rayons ultraviolets, ladite au moins une source de rayons ultraviolets pouvant être au moins une diode électroluminescente 19 émettant des rayonnements de la longueur d'onde des ultraviolets de type A. Les molécules organiques odorantes sont alors minéralisées.

Le dispositif de traitement des odeurs 16 est efficace tout au long de la durée de vie de la machine à laver et à sécher la vaisselle 1 sans avoir à changer un élément ou à recharger un produit.

Le dispositif de traitement des odeurs 16 placé à l'intérieur de la machine à laver et à sécher la vaisselle 1 permet de capter et de traiter rapidement et efficacement les mauvaises odeurs enfermées dans ladite machine 1.

Le dispositif de traitement des odeurs 16 peut fonctionner périodiquement entre les cycles de lavage de la machine à laver et à sécher la vaisselle 1 pendant une durée prédéterminée ou suivant une durée calculée par un moyen de commande (non représenté) de ladite machine 1, ou encore suivant une commande ordonnée par un utilisateur.

Selon une caractéristique préférée de l'invention, le dispositif de traitement des odeurs 16 est placé à l'intérieur d'un conduit de ventilation 11 du circuit de séchage 3 de ladite machine 1.

Ainsi, les odeurs contenues dans la machine à laver et à sécher la vaisselle 1 sont aspirées et circulent à travers un conduit de ventilation 11. La circulation d'air à l'intérieur de la machine à laver et à sécher la vaisselle 1 est réalisée par au moins un ventilateur 12. Ladite circulation d'air de la machine à laver et à sécher la vaisselle 1 est dite forcée.

Le dispositif de traitement des odeurs 16 est placé à l'intérieur d'un conduit de ventilation 11 déjà existant sur les machines à laver et à sécher la vaisselle classiques utilisant un mode de séchage par convection forcée. De cette manière, le dispositif de traitement des odeurs 16 peut être intégré à une machine classique sans modifications structurelles engendrant un coût supplémentaire.

Le circuit de ventilation 3 de la machine à laver et à sécher la vaisselle 1 peut être fermé ou encore légèrement ouvert pour permettre une entrée d'air extérieur.

Le dispositif de traitement des odeurs 16 est inséré dans un conduit de ventilation 11 du circuit de séchage 3 de la machine à laver et à sécher la vaisselle 1 sans créer de perturbations aérauliques. L'implantation dispositif de traitement des odeurs 16 dans un conduit de ventilation 11 du circuit de séchage 3 de la machine à laver et à sécher la vaisselle 1 ne génère pas de pertes de charges supplémentaires.

Entre deux cycles de lavage, le dispositif de traitement des odeurs 16 peut fonctionner en simultané avec ledit au moins un ventilateur 12 de la machine à laver et à sécher la vaisselle 1. Ledit au moins un ventilateur 12 assurant la circulation interne d'air en traversant la cuve de lavage 2 peut fonctionner en continu ou en discontinu lors d'un cycle de désodorisation de la machine à laver et à sécher le linge 1.

Le dispositif de traitement des odeurs 16 doit fonctionner régulièrement pour empêcher que la vaisselle sale rangée dans la cuve de lavage 2 de la machine à laver et à sécher la vaisselle 2 ne dégage pas de mauvaises odeurs.

Le dispositif de traitement des odeurs 16 peut fonctionner aux périodes précédant les repas, notamment aux périodes où les utilisateurs remplissent leur machine à laver et à sécher la vaisselle 1. Ces périodes nécessitent une destruction efficace des odeurs avant d'ouvrir la porte de la machine à laver et à sécher la vaisselle 1. La durée de fonctionnement du dispositif de traitement des odeurs 16 peut être de l'ordre de 30 minutes à deux heures en continu, et préférentiellement de l'ordre d'une heure et demie.

Selon une autre caractéristique préférée de l'invention, ledit au moins un catalyseur 18 est du dioxyde de titane.

Ledit au moins un catalyseur 18 permet le fonctionnement du dispositif de traitement des odeurs 16 en étant mouillé.

Préférentiellement, ledit au moins un catalyseur 16 est déposé sur un support 20 non tissé de forme plane.

De cette manière, le support 20 rentre aisément dans un conduit de ventilation 11 de la machine à laver et à sécher la vaisselle 1 sans modification de ladite machine 1 et en ne créant pas de pertes de charges supplémentaires.

Ce support 20 permet d'utiliser le dispositif de traitement des odeurs 16 tout en étant mouillé régulièrement et en ne se dégradant pas au cours de la durée de vie de la machine à laver et à sécher la vaisselle 1.

Selon une autre caractéristique préférée de l'invention, un flux d'air à l'intérieur du conduit de ventilation 11 est tangentiel au support 20 dudit au moins un catalyseur 18.

Ainsi, le flux d'air F à l'intérieur du conduit de ventilation 11 lèche au moins une surface dudit au moins un catalyseur 18 pouvant être irradiée au même instant par ladite au moins une source de rayons ultraviolets 19. Dans ce mode de réalisation de l'invention, le flux d'air F du conduit de ventilation 11 ne traverse pas le support 20 dudit au moins un catalyseur 18.

Cette disposition du support 20 dudit au moins un catalyseur 18 dans le conduit de ventilation 11 empêche une nuisance sonore plus forte que celle engendrée par ledit au moins un ventilateur 12.

Selon une autre caractéristique préférée de l'invention, le dispositif de traitement des odeurs 16 comprend au moins un catalyseur 18 associé à une couche de charbon actif 21.

Ainsi, le support 20 du dispositif de traitement des odeurs 16 comprend au moins une couche d'au moins un catalyseur 18 tel que le dioxyde de titane et au moins une couche de charbon actif 21. Ladite au moins une couche de charbon actif 21 est de servir de tampon et d'adsorber les pics importants d'odeurs. Le charbon actif 21 est simultanément régénéré par ladite au moins une couche de catalyseur 18 sous l'irradiation de ladite au moins une source de rayons ultraviolets 19.

Une partie des molécules organiques odorantes sont piégées dans une couche de charbon actif 21 jouant le rôle de tampon et une autre partie des molécules organiques odorantes entrent en contact avec une surface dudit au moins un catalyseur 18 irradiée par ladite au moins une source de rayons ultraviolets 19 pour être minéralisées. Lesdites molécules organiques odorantes piégées dans la couche de charbon actif 21 se déplacent vers la couche dudit au moins un catalyseur 18 dès que l'autre partie des molécules organiques odorantes sont détruites.

Ladite au moins une couche de charbon actif 21 doit être préférentiellement sèche pour adsorber les molécules organiques odorantes.

Dans le cas où le charbon actif 21 est mouillé, ce dernier peut être séché par ledit au moins un ventilateur 12 du circuit de séchage 3 de la machine à laver et à sécher la vaisselle 1.

Préférentiellement, le rayonnement de ladite au moins une source de rayons ultraviolets 19 sur ledit au moins un catalyseur 18 s'effectue au travers d'une paroi 15 transparente aux rayonnements ultraviolets du conduit de ventilation 11.

Ainsi, le positionnement de ladite au moins une source de rayons ultraviolets 19 permet d'obtenir un rayonnement à travers un matériau transparent aux rayons ultraviolets. Ladite au moins une source de rayons ultraviolets 19 est isolée du circuit de ventilation 3 et de l'humidité. Le matériau de la paroi 15 transparente aux rayonnements ultraviolets du conduit de ventilation 11 peut être un polypropylène transparent laissant passer au moins 60% des rayons ultraviolets de type A pour une épaisseur de l'ordre de 1,5mm.

L'épaisseur de la paroi 15 transparent aux rayonnements ultraviolets du conduit de ventilation 11 peut être diminuée afin d'augmenter l'irradiation dudit au moins un catalyseur 18. La diminution d'épaisseur de la paroi 15 transparent aux rayonnements ultraviolets du conduit de ventilation 11 peut être localisée au niveau d'au moins un emplacement placé en vis-à-vis d'au moins une source de rayons ultraviolets 19.

En pratique, l'épaisseur de la paroi 15 transparent aux rayonnements ultraviolets du conduit de ventilation 11 peut être comprise dans une plage s'étendant entre 0,5mm et 2mm, et préférentiellement de l'ordre de 0,8mm.

En pratique, ladite au moins une source de rayons ultraviolets 19 est placée à l'extérieur du conduit de ventilation 11.

Dans un autre mode de réalisation de l'invention, ladite au moins une diode source de rayons ultraviolets 19 peut être positionnée de manière à avoir un rayonnement direct sur le support 20 du dispositif de traitement des odeurs 16 comprenant au moins un catalyseur 18. Dans ce cas, ladite au moins une source de rayons ultraviolets 19 est en contact direct avec l'humidité résiduelle du circuit de ventilation 3 de la machine à laver et à sécher la vaisselle 1. D'où la nécessité de protéger électriquement ladite au moins une source de rayons ultraviolets 19 et les moyens de commande de cette dernière par exemple par une couche de résine. Les moyens de commande de ladite au moins une source de rayons ultraviolets peuvent être au moins une carte électronique 22.

Selon une autre caractéristique préférée de l'invention, le support 20 dudit au moins un catalyseur 18 est placé à distance d'une paroi inférieure 14 et d'une paroi supérieure 15 du conduit de ventilation 11.

Ainsi, le support 20 comprenant ledit au moins un catalyseur 18 est positionné au travers du flux d'air F du conduit de ventilation 11. De cette manière, les molécules organiques odorantes circulent sur les deux faces dudit support 20 non tissé et de forme plane. En outre, la distance séparant ledit support 20 de ladite au moins une source de rayons ultraviolets 19 est réduite et permet une irradiation dudit au moins un catalyseur 18 plus puissante.

Le dispositif de traitement des odeurs 16 peut être activé par une touche (non représentée) pour un fonctionnement manuel ou automatique. Cette touche permet également de débrayer le fonctionnement du dispositif de traitement des odeurs 16.

On va décrie à présent un premier mode de réalisation de l'invention illustré aux figures 2 à 4.

Le dispositif de traitement des odeurs 16 comprend un carter 23 fixé à une paroi supérieure 15 du conduit de ventilation 11 par des moyens de fixation 24 tels que des vis de fixation.

Une carte électronique de commande 22 du dispositif de traitement des odeurs 16 est placée entre la paroi supérieure 15 du conduit de ventilation 11 et le carter 23 du dispositif de traitement des odeurs 16.

Le carter 23 du dispositif de traitement des odeurs 16 comprend au moins une ouverture 25 pour permettre le passage d'un câble de connexion de la carte électronique de commande 22.

La carte électronique de commande 22 du dispositif de traitement des odeurs 16 comprend une pluralité de diodes électroluminescentes 19 pour irradier la surface recouverte d'au moins un catalyseur 18.

Ladite au moins une source de rayons ultraviolets 19 émet à une longueur d'onde de l'ordre de 365nm ou encore à une valeur de l'ordre de 375nm, et préférentiellement inférieure à 380nm.

Le nombre de diodes électroluminescentes 19 utilisées est donné à titre d'exemple non limitatif. Une seule diode électroluminescente 19 peut également être utilisée. On peut également utiliser plus ou moins six diodes.

Préférentiellement, le dispositif de traitement des odeurs 16 comprend quatre diodes électroluminescentes 19 pour supprimer les odeurs renfermées dans la machine à laver et à sécher la vaisselle 1.

Ledit au moins un catalyseur 18 est maintenu par au moins un support 20 placé entre la paroi supérieure 15 et la paroi inférieure 14 du conduit de ventilation 11. Le support 20 dudit au moins un catalyseur 18 comprend des moyens de fixation 26 tels que des moyens d'encliquetage élastique coopérant avec la paroi supérieure 15 et / ou la paroi inférieure 14 du conduit de ventilation 11 et / ou le carter 23 du dispositif de traitement des odeurs 16.

Ledit au moins un catalyseur 18 peut être sous la forme d'un papier, ou encore appelé « non-tissé ».

Lesdites diodes électroluminescentes 19 sont situées au-dessus de la paroi supérieure 15 du conduit de ventilation 14 de manière à irradier la surface dudit au moins un catalyseur 18.

Le flux d'air F à l'intérieur du conduit de ventilation 11 passe de part et d'autre du support 20 maintenant ledit au moins un catalyseur 18.

Le support 20 dudit au moins un catalyseur 18 peut avoir des dimensions de l'ordre 75mm de longueur, de 65mm de largeur et de 2mm d'épaisseur.

La surface dudit au moins un catalyseur 18 irradiée par ladite au moins une source de rayons ultraviolets 19 peut s'étendre sur une longueur de 70mm pour une largeur de l'ordre de 60mm.

Le support 20 dudit au moins un catalyseur 18 peut être réalisé en surmoulage de manière à réaliser un filtre simple à installer dans le conduit de ventilation 11.

La distance entre ladite au moins une source de rayons ultraviolets 19 et la surface dudit au moins un catalyseur 18 irradiée peut être comprise entre 4mm et 20mm, et préférentiellement de l'ordre de 12mm.

La distance entre ladite au moins une source de rayons ultraviolets 19 et ledit au moins un catalyseur 18 est déterminée de manière à couvrir la plus grande surface irradiée dudit au moins un catalyseur 18.

La diffusion de rayonnements ultraviolets par ladite au moins une source de rayons ultraviolets 19 est de forme conique.

Ladite distance est déterminée pour obtenir une surface d'irradiation dudit au moins un catalyseur 18 avec une intensité lumineuse optimales. Un compromis est déterminé afin d'obtenir des résultats optimums en ayant un minimum de diodes électroluminescentes 19 pour irradier ledit au moins un catalyseur 18.

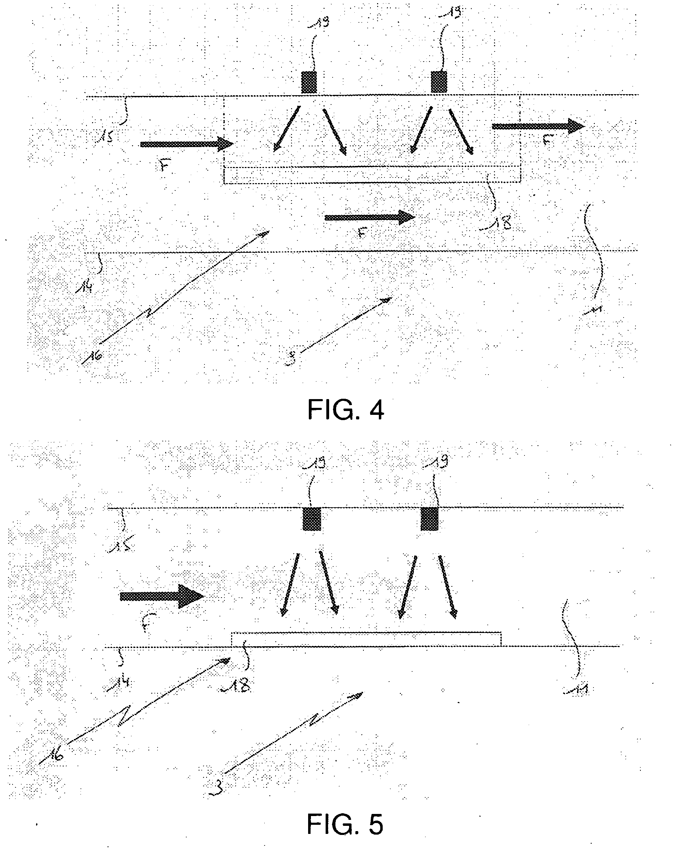

Dans un autre mode de réalisation de l'invention illustré à la figure 5, ledit au moins un catalyseur 18 est placé contre la paroi inférieure 14 du conduit de ventilation 11. Ladite au moins une source de rayons ultraviolets 19 peut être placée à l'intérieur du conduit de ventilation 11 pour garantir une irradiation satisfaisante dudit au moins un catalyseur 18. Dans cette configuration, le flux d'air F à l'intérieur du conduit de ventilation 11 ne lèche qu'une surface dudit au moins un catalyseur 18. Bien entendu, ledit au moins un catalyseur 18 peut être associé à une couche de charbon actif 21. Ainsi, le dispositif de traitement des odeurs 16 permet de capter les molécules organiques odorantes avant que ces dernières ne soient détruites afin de faire tampon au niveau des pics d'odeurs.

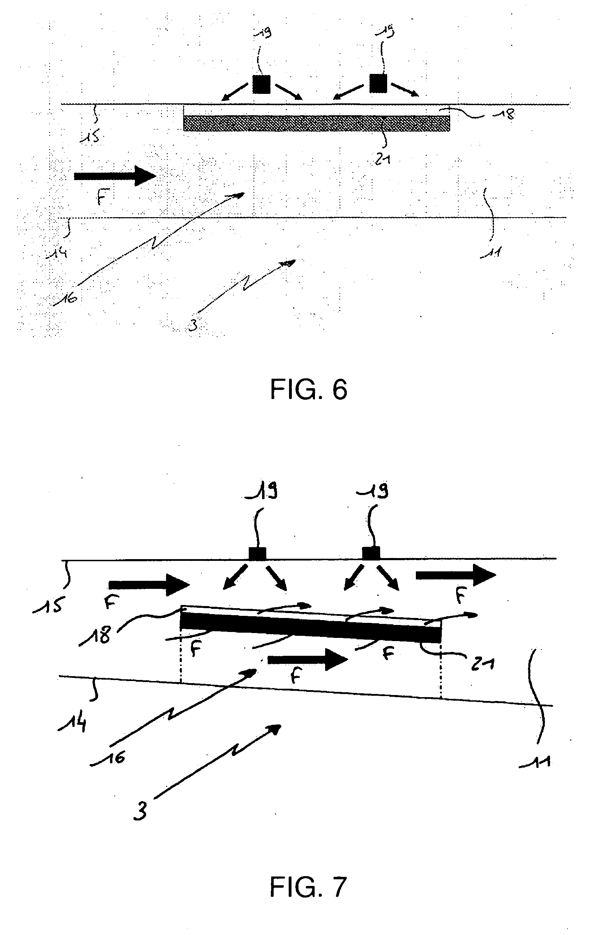

Dans un autre mode de réalisation de l'invention illustré à la figure 6, ledit au moins un catalyseur 18 est placé contre la paroi supérieure 15 du conduit de ventilation 11. Ladite au moins une source de rayons ultraviolets 19 peut être placée à l'extérieur du conduit de ventilation 11 pour garantir une irradiation satisfaisante dudit au moins un catalyseur 18. Les rayonnements ultraviolets de ladite au moins une source de rayons ultraviolets 19 traversent une paroi 15 transparente aux rayonnements ultraviolets du conduit de ventilation. Ladite au moins une source de rayons ultraviolets 19 et la surface irradiée dudit au moins un catalyseur 18 sont à proximité. Dans cette configuration, le flux d'air F à l'intérieur du conduit de ventilation 11 ne lèche qu'une surface dudit au moins un catalyseur 11. Bien entendu, ledit au moins un catalyseur 18 peut être associé à une couche de charbon actif 21. Ainsi, le dispositif de traitement des odeurs 16 permet de capter les molécules organiques odorantes avant que ces dernières ne soient détruites afin de faire tampon au niveau des pics d'odeurs. La couche dudit au moins un catalyseur 18 est placée entre la paroi supérieure 15 du conduit de ventilation 11 et la couche de charbon actif 21.

Dans un autre mode de réalisation de l'invention illustré à la figure 7, ledit au moins un catalyseur 18 est placé à distance de la paroi supérieure 15 et la paroi inférieure 14 du conduit de ventilation 11. Ladite au moins une source de rayons ultraviolets 19 peut être placée à l'extérieur du conduit de ventilation 11 pour garantir une irradiation satisfaisante dudit au moins un catalyseur 18. Les rayonnements ultraviolets de ladite au moins une source de rayons ultraviolets 19 traversent une paroi 15 transparente aux rayonnements ultraviolets du conduit de ventilation. Le flux d'air F à l'intérieur du conduit de ventilation 11 passe de part et d'autre du support 20 maintenant ledit au moins un catalyseur 18. En outre, le dispositif de traitement des odeurs 16 peut être incliné par rapport au conduit de ventilation 11. Ainsi, le flux d'air F traverse en partie ledit au moins un catalyseur 18. L'inclinaison dudit dispositif de traitement des odeurs 16 peut être comprise entre 2° et 15°. Bien entendu, ledit au moins un catalyseur 18 peut être associé à une couche de charbon actif 21. Ainsi, le dispositif de traitement des odeurs 16 permet de capter les molécules organiques odorantes avant que ces dernières ne soient détruites afin de faire tampon au niveau des pics d'odeurs. Le flux d'air F traverse également la couche de charbon actif 21. Les molécules organiques odorantes piégées dans le charbon actif 21 sont poussées en direction de la couche dudit au moins un catalyseur 18 par le flux d'air F généré par ledit au moins un ventilateur 12. Lorsque les molécules organiques odorantes arrivent en contact avec ledit au moins un catalyseur 18, elles sont traitées par photocatalyse grâce à ladite au moins une source de rayons ultraviolets 19. Relativement au sens de circulation du flux d'air F, la couche dudit au moins un catalyseur 18 est disposée sur une face aval du support 20. Par ailleurs, la paroi inférieure 14 du conduit de ventilation 11 peut être inclinée. L'inclinaison de la paroi inférieure 14 du conduit de ventilation 11 peut être sensiblement identique à celle du dispositif de traitement des odeurs 16.

On va décrire à présent un procédé de traitement des odeurs dans une machine à laver et à sécher la vaisselle conforme à l'invention.

Ladite machine à laver et à sécher la vaisselle 1 comprend une cuve de lavage 2 ayant une paroi de fond 6, des parois latérales 10 et une paroi supérieure 5 formant une cavité 2 pour le lavage de la vaisselle, dont la face frontale 17 de la cavité 2 est fermée par une porte.

Ladite machine à laver et à sécher la vaisselle 1 comprend également un dispositif de traitement des odeurs 16, ledit dispositif de traitement des odeurs 16 comprenant au moins un catalyseur 18, et ledit au moins un catalyseur 18 étant irradié par au moins une source de rayons ultraviolets 19.

Le dispositif de traitement des odeurs 16 fonctionne périodiquement.

Ainsi, le dispositif de traitement des odeurs 16 fonctionne par cadence pour réduire les coûts liés à la durée de vie des composants et éviter tout remplacement d'un élément de la machine 1.

La machine à laver et à sécher la vaisselle 1 est conçue en prédéterminant :
- une durée de fonctionnement dudit au moins un ventilateur 12 pour faire circuler les odeurs dans la machine 1 et en particulier dans le conduit de ventilation 11 comprenant le dispositif de traitement des odeurs 16,
- une durée d'allumage de ladite au moins une source de rayons ultraviolets 19 pour supprimer les odeurs par traitement photocatalytique, ledit au moins un ventilateur 12 est mis en fonctionnement au cours de cette étape pour faire circuler l'air dans le conduit de ventilation 11, la durée d'allumage de ladite au moins une source de rayons ultraviolets 19 permet également de régénérer le charbon actif 21, et
- une durée d'arrêt du dispositif de traitement des odeurs 16 et dudit au moins un ventilateur 12 de la machine à laver et à sécher la vaisselle 1.

Le temps d'arrêt du dispositif de traitement des odeurs 16 et dudit au moins un ventilateur 12 doit être d'une durée inférieure à la durée de propagation des odeurs de vaisselle sale dans la machine à laver et à sécher la vaisselle 1. En effet, les salissures présentes sur la vaisselle renouvellent les mauvaises odeurs dans la cuve de lavage 2 rapidement. La demanderesse a pu constater que la machine à laver et à sécher la vaisselle 1 est de nouveau polluée au bout de quelques heures.

Préférentiellement, le dispositif de traitement des odeurs 16 se déclenche après chaque ouverture de la porte de la machine 1.

Chaque ouverture de porte de la machine à laver et à sécher la vaisselle 1 correspond généralement à une introduction de vaisselle sale supplémentaire.

La durée de traitement des odeurs est comprise dans une plage s'étendant entre 4 minutes et 20 minutes par heure.

Le débit d'air généré par ledit au moins un ventilateur 12 de la machine à laver et à sécher la vaisselle 1 est compris dans une plage s'étendant entre 3 et 7m³/h et préférentiellement de l'ordre de 5m³/h. De cette manière, le volume d'air compris dans la cuve de lavage 2 est traité par le dispositif de traitement des odeurs 16.

La vitesse du flux d'air F à l'intérieur du conduit de ventilation 11 est de l'ordre de 1,3m/s dans le mode de réalisation de l'invention décrit précédemment. La vitesse du flux d'air F à l'intérieur du conduit de ventilation 11 peut être comprise entre 0,2m/s et 10m/s, et de préférence de l'ordre de 1 m/s.

Sur une période d'une heure, la durée de fonctionnement dudit au moins un ventilateur 12 est de l'ordre de deux minutes pour que l'ensemble de l'air de la cuve de lavage 2 circule dans le conduit de ventilation 11 comprenant le dispositif de traitement des odeurs 16. Le volume de la cuve de lavage 2 étant de l'ordre de 150 litres.

Le dispositif de traitement des odeurs 16 permet de détruire les mauvaises odeurs en réalisant une circulation d'air contenue dans la machine à laver et à sécher la vaisselle 1 depuis la cuve de lavage 2 vers le circuit de ventilation 3. Cette circulation d'air est mise en oeuvre par ledit au moins un ventilateur 12 et permet un bouclage de l'air dans la machine 1. Le bouclage de la circulation d'air permet de traiter les odeurs par des passages successifs des molécules organiques odorantes sur le dispositif de traitement des odeurs 16.

Pour effectuer un traitement des odeurs satisfaisant, la Demanderesse a pu constater que cinq passages complets de l'air contenu dans la cuve de lavage 2 sont nécessaires pour retirer les odeurs de la machine à laver et à sécher la vaisselle 1. Ce qui représente un temps de fonctionnement du dispositif de traitement des odeurs 16 et dudit au moins un ventilateur 12 de l'ordre de 10 minutes. Le rapport de marche du dispositif de traitement des odeurs 16 est alors de l'ordre de 17%.

Le temps de fonctionnement du dispositif de traitement des odeurs 16 et dudit au moins un ventilateur 12 peut être rallongé à 15 minutes pour s'affranchir des ouvertures répétées de la porte de la machine à laver et à sécher la vaisselle 1 lors de l'introduction de vaisselle sale dans les paniers de chargement.

Le dispositif de traitement des odeurs 16 se déclenche de manière cyclique en dehors des cycles de lavage de ladite machine 1.

Un cycle complet de fonctionnement du dispositif de traitement des odeurs 16 peut être compris dans une heure où une période de traitement par photocatalyse a une durée de 10 minutes. Ce cycle peut être répété plusieurs fois.

Lorsque la machine à laver et à sécher la vaisselle 1 est dans une période où aucun cycle de lavage est ordonné par les moyens de commande, le dispositif de traitement des odeurs 16 et ledit au moins un ventilateur 12 peut fonctionner de manière cyclique suivant une durée de fonctionnement de l'ordre de 10 minutes pour une durée d'arrêt de 50 minutes.

Le premier cycle de traitement des odeurs après une ouverture de porte de la machine à laver et à sécher la vaisselle 1 peut comprendre une durée de traitement par photocatalyse de l'ordre de 15 minutes puis les cycles suivants sont enchaînés sur une durée de traitement par photocatalyse d'une durée de l'ordre de 10 minutes pour une durée d'arrêt du dispositif de traitement des odeurs 16 de l'ordre de 50 minutes.

Le nombre de passages de l'air dans le conduit de ventilation 11 comprenant le dispositif de traitement des odeurs 16 est compris entre 1 et 15 en fonction du débit d'air généré par ledit au moins un ventilateur 12.

Le dispositif de traitement des odeurs 16 se met en veille après une période prédéterminée de non ouverture de la porte de ladite machine 1.

Dans ce cas, l'utilisateur est considéré comme absent pour une période longue et à la prochaine ouverture de porte le dispositif de traitement des odeurs 16 se réveille comme décrit précédemment.

La période prédéterminée de non ouverture de la porte de ladite machine 1 peut être de l'ordre de 5 jours.

La machine à laver et à sécher la vaisselle 1 comprend au moins une ouverture d'entrée d'air 8 dans une paroi 5 de la cuve de lavage 2 permettant l'aspiration des odeurs par au moins un ventilateur 12, lesdites odeurs circulant dans un conduit de ventilation 11 et traversant le dispositif de traitement des odeurs 16 par photocatalyse, puis l'air traité par ledit dispositif de traitement des odeurs 16 par photocatalyse étant évacué au travers d'au moins une ouverture de sortie 9 dans une paroi de 10 la cuve de lavage 2.

Ladite au moins une ouverture d'entrée d'air 8 est ménagée dans la paroi supérieure 5 de la cuve de lavage 2. Ladite au moins une ouverture d'entrée d'air 8 est directement reliée à une zone d'aspiration d'au moins un ventilateur 12. Ledit au moins un ventilateur 12 est dit « centrifuge » ou encore « axial radial ». La zone de soufflage dudit au moins un ventilateur 12 est reliée à une première extrémité du conduit de ventilation 11 du circuit d'air de séchage 3 de la machine à laver et à sécher la vaisselle 1. Ce conduit de ventilation 11 ménagé dans le dessus 5 de cuve de lavage 2 tel qu'illustré à la figure 1 comprend le dispositif de traitement des odeurs 16. La seconde extrémité du conduit de ventilation 11 est soit directement reliée à ladite au moins une ouverture de sortie d'air 9 ménagée dans une paroi latérale 10 de la cuve de lavage 2, soit reliée à un condenseur 13 étant lui-même relié à ladite au moins une ouverture de sortie d'air 9 également ménagée dans une paroi latérale 10 de la cuve de lavage 2.

Ledit au moins un ventilateur 12 situé dans le conduit de ventilation 11 de la machine à laver et à sécher la vaisselle 1 est en amont du dispositif de traitement des odeurs 16.

Lesdites au moins une ouverture d'entrée d'air 8 et au moins une ouverture de sortie d'air 9 ainsi que le circuit de ventilation 3 sont disposés dans la partie arrière de ladite machine 1.

Ledit au moins un ventilateur 12 et ladite au moins une source de rayons ultraviolets 19 fonctionnent simultanément pour le traitement des odeurs.

Ladite au moins une source de rayons ultraviolets 19 fonctionne pendant une durée supplémentaire à une durée de traitement des odeurs pour régénérer une couche de charbon actif 21 déposée sur un support 20 comprenant ledit au moins un catalyseur 18.

La régénération dudit charbon actif 21 est réalisée sans la mise en fonctionnement dudit au moins un ventilateur 12.

La durée de fonctionnement du dispositif de traitement des odeurs 16 pour détruire les odeurs et la durée de fonctionnement supplémentaire pour régénérer ladite couche de charbon actif 21 peuvent être réalisées en continu. Ainsi, ladite au moins une source de rayons ultraviolets 19 est allumée en continu.

Une partie des molécules organiques odorantes sont piégées dans une couche de charbon actif 21 jouant un rôle de tampon et l'autre partie des molécules organiques odorantes entrent en contact avec une surface dudit au moins un catalyseur 18 irradiée par au moins une source de rayons ultraviolets de type A. Cette dernière partie des molécules organiques odorantes sont alors minéralisées. Les molécules organiques odorantes piégées dans la couche de charbon actif 21 se déplacent vers la surface dudit au moins un catalyseur 18 dès que les molécules organiques odorantes précédentes sont détruites.

Ladite au moins une source de rayons ultraviolets 19 fonctionne pendant une durée supplémentaire pour régénérer une couche de charbon actif 21 déposée sur un support 20 comprenant ledit au moins un catalyseur 18 comprise dans une plage s'étendant entre 4 minutes et 10 minutes, et préférentiellement de l'ordre de 8 minutes.

L'eau provenant de la décomposition des molécules organiques odorantes est recueillie soit dans un bac soit par une crépine. La récupération d'eau provenant du dispositif de traitement des odeurs 16 est identique à celle de l'eau de condensation pendant une phase de séchage de la vaisselle de la machine à laver et à sécher la vaisselle 1.

Bien entendu, de nombreuses modifications peuvent être apportées aux exemples de réalisation ci-dessus sans sortir du cadre de l'invention.

## Revendications

1. Machine à laver et à sécher la vaisselle comprenant une cuve de lavage (2) ayant une paroi de fond (6), des parois latérales (10) et une paroi supérieure (5) formant une cavité (2) pour le lavage de la vaisselle, dont la face frontale (17) de la cavité (2) est fermée par une porte, un dispositif de traitement des odeurs (16), ledit dispositif de traitement des odeurs (16) comprenant au moins un catalyseur (18) associé à une couche de charbon actif (21), et ledit au moins un catalyseur (18) étant irradié par au moins une source de rayons ultraviolets (19), ledit dispositif de traitement des odeurs (16) étant placé à l'intérieur d'un conduit de ventilation (11) du circuit de séchage (3) de ladite machine (1).

2. Machine à laver et à sécher la vaisselle selon la revendication 1, **caractérisée en ce que** ledit au moins un catalyseur (18) est du dioxyde de titane.

3. Machine à laver et à sécher la vaisselle selon la revendication 1 ou 2, **caractérisée en ce que** ledit au moins un catalyseur (18) est déposé sur un support (20) non tissé de forme plane.

4. Machine à laver et à sécher la vaisselle selon la revendication 3, **caractérisée en ce qu'**un flux d'air (F) à l'intérieur du conduit de ventilation (11) est tangentiel au support (20) dudit au moins un catalyseur (18).

5. Machine à laver et à sécher la vaisselle selon l'une quelconque des revendications 2 à 4, **caractérisée en ce que** le rayonnement de ladite au moins une source de rayons ultraviolets (19) sur ledit au moins un catalyseur (18) s'effectue au travers d'une paroi transparente (15) aux rayonnements ultraviolets du conduit de ventilation (11).

6. Machine à laver et à sécher la vaisselle selon l'une quelconque des revendications 2 à 5, **caractérisée en ce que** ladite au moins une source de rayons ultraviolets (19) est placée à l'extérieur du conduit de ventilation (11).

7. Machine à laver et à sécher la vaisselle selon l'une quelconque des revendications 4 à 6, **caractérisée en ce que** le support (20) dudit au moins un catalyseur (18) est placé à distance d'une paroi inférieure (14) et d'une paroi supérieure (15) du conduit de ventilation (11).

8. Procédé de traitement des odeurs dans une machine à laver et à sécher la vaisselle conforme à l'une quelconque des revendications 1 à 7, **caractérisée en ce que** le dispositif de traitement des odeurs (16) fonctionne périodiquement.

9. Procédé de traitement des odeurs dans une machine à laver et à sécher la vaisselle selon la revendication 8, **caractérisé en ce que** le dispositif de traitement des odeurs (16) se déclenche après chaque ouverture de la porte de la machine (1).

10. Procédé de traitement des odeurs dans une machine à laver et à sécher la vaisselle selon la revendication 8 ou 9, **caractérisé en ce que** le dispositif de traitement des odeurs (16) se déclenche de manière cyclique en dehors des cycles de lavage de ladite machine (1).

11. Procédé de traitement des odeurs dans une machine à laver et à sécher la vaisselle selon l'une quelconque des revendication 8 à 10, **caractérisé en ce que** le dispositif de traitement des odeurs (16) se met en veille après une période prédéterminée de non ouverture de la porte de ladite machine (1).

12. Procédé de traitement des odeurs dans une machine à laver et à sécher la vaisselle selon l'une quelconque des revendication 8 à 11, **caractérisé en ce que** la machine à laver et à sécher la vaisselle (1) comprend au moins une ouverture d'entrée d'air (8) dans une paroi (5) de la cuve de lavage (2) permettant l'aspiration des odeurs par au moins un ventilateur (12), lesdites odeurs circulant dans un conduit de ventilation (11) et traversant le dispositif de traitement des odeurs (16) par photocatalyse, puis l'air traité par ledit dispositif de traitement des odeurs (16) par photocatalyse étant évacué au travers d'au moins une ouverture de sortie (9) dans une paroi (10) de la cuve de lavage (2).

13. Procédé de traitement des odeurs dans une machine à laver et à sécher la vaisselle selon l'une quelconque des revendication 8 à 12, **caractérisé en ce que** la durée de traitement des odeurs est comprise dans une plage s'étendant entre 4 minutes et 20 minutes par heure.

14. Procédé de traitement des odeurs dans une machine à laver et à sécher la vaisselle selon l'une quelconque des revendication 12 à 13, **caractérisé en ce que** ledit au moins un ventilateur (12) et ladite au moins une source de rayons ultraviolets (19) fonctionnent simultanément pour le traitement des odeurs.

15. Procédé de traitement des odeurs dans une machine à laver et à sécher la vaisselle selon l'une quelconque des revendication 8 à 14, **caractérisé en ce que** ladite au moins une source de rayons ultraviolets (19) fonctionne pendant une durée supplémentaire à une durée de traitement des odeurs pour régénérer une couche de charbon actif (21) déposée sur un support (20) comprenant ledit au moins un catalyseur (18).

16. Procédé de traitement des odeurs dans une machine à laver et à sécher la vaisselle selon la revendication 15, **caractérisé en ce que** la régénération dudit charbon actif (21) est réalisée sans la mise en fonctionnement dudit au moins un ventilateur (12).

## Claims

1. A machine for washing and drying dishes comprising a wash tub (2) having a bottom wall (6), side walls (10) and an upper wall (5) forming a dish-washing cavity (2), the front face (17) of said cavity (2) being shut by a door, and an odor treatment device (16), said odor treatment device (16) comprising at least one catalyst (18) associated with an activated carbon layer (21), and said at least one catalyst (18) being irradiated by at least one source of ultraviolet radiation (19), said odor treatment device (16) being placed inside a ventilation duct (11) of said machine's (1) drying circuit (3).

2. A machine for washing and drying dishes according to claim 1, **characterized in that** said at least one catalyst (18) is made of titanium dioxide.

3. A machine for washing and drying dishes according to claim 1 or 2, **characterized in that** said at least one catalyst (18) is deposited onto a non-woven substrate (20) that is flat in shape.

4. A machine for washing and drying dishes according to claim 3, **characterized in that** an air flow (F) within the ventilation duct (11) is tangential to the substrate (20) of said at least one catalyst (18).

5. A machine for washing and drying dishes according to one of the claims 2 to 4, **characterized in that** the radiation from said at least one source of ultraviolet radiation (19) onto said at least one catalyst (18) travels through an ultraviolet-transparent wall (15) of the ventilation duct (11).

6. A machine for washing and drying dishes according to one of the claims 2 to 5, **characterized in that** said at least one source of ultraviolet radiation (19) is placed outside the ventilation duct (11).

7. A machine for washing and drying dishes according to one of the claims 4 to 6, **characterized in that** the substrate (20) of said at least one catalyst (18) is placed a distance away from a lower wall (14) and from an upper wall (15) of the ventilation duct (11).

8. A method for treating odors in a machine for washing and drying dishes according to one of the claims 1 to 7, **characterized in that** the odor treatment device (16) operates periodically.

9. A method for treating odors in a machine for washing and drying dishes according to claim 8, **characterized in that** the odor treatment device (16) triggers after each opening of the door of the machine (1),

10. A method for treating odors in a machine for washing and drying dishes according to claim 8 or 9, **characterized in that** the odor treatment device (16) triggers cyclically outside of said machine's (1) wash cycles.

11. A method for treating odors in a machine for washing and drying dishes according to one of the claims 8 to 10, **characterized in that** the odor treatment device (16) enters standby mode after a preset period during which the door of said machine (1) is not opened.

12. A method for treating odors in a machine for washing and drying dishes according to one of the claims 8 to 11, **characterized in that** the machine for washing and drying dishes (1) comprises at least one air inlet (8) within a wall (5) of the wash tub (2) enabling the suction of odors by at least one fan (12), said odors traveling through a ventilation duct (11) and passing through the odor treatment device (16) via photocatalysis, followed by the air treated by said odor treatment device (16) via photocatalysis being discharged through at least one outlet (9) within a wall (10) of the wash tub (2).

13. A method for treating odors in a machine for washing and drying dishes according to one of the claims 8 to 12, **characterized in that** the duration of the odor treatment falls within a range between 4 minutes and 20 minutes per hour.

14. A method for treating odors in a machine for washing and drying dishes according to one of the claims 12 to 13, **characterized in that** said at least one fan (12) and said at least one source of ultraviolet radiation (19) operate simultaneously to treat odors.

15. A method for treating odors in a machine for washing and drying dishes according to one of the claims 8 to 14, **characterized in that** said at least one source of ultraviolet radiation (19) operates for an additional period beyond an odor treatment period in order to regenerate the activated carbon layer (21) deposited onto a substrate (20) comprising said at least one catalyst (18).

16. A method for treating odors in a machine for washing and drying dishes according to claim 15, **characterized in that** the regeneration of said activated carbon layer (21) is performed without turning on said at least one fan (12).

## Patentansprüche

1. Geschirrspüler/-trockner, ausgestattet mit einem Spültank (2) mit einer Bodenwand (6), Seitenwänden (10) und einer oberen Wand (5), welche einen Tank (2) zum Spülen des Geschirrs bilden, wobei die Stirnfläche (17) des Tanks (2) durch eine Tür geschlossen wird, einer Vorrichtung zur Geruchsbehandlung (16), wobei die besagte Vorrichtung zur Geruchsbehandlung (16) mindestens einen mit einer Aktivkohleschicht (21) assoziierten Katalysator (18) umfasst, und wobei der besagte mindestens eine Katalysator (18) von mindestens einer Ultraviolettstrahlenquelle (19) bestrahlt wird, wobei die besagte Vorrichtung zur Geruchsbehandlung (16) im Inneren eines Belüftungskanals (11) des Trocknungskreislaufs (3) der besagten Maschine (1) angeordnet ist.

2. Geschirrspüler/-trockner nach Anspruch 1, **dadurch gekennzeichnet, dass** der besagte mindestens eine Katalysator (18) ein Titandioxid ist.

3. Geschirrspüler/-trockner nach Anspruch 1 oder 2, **dadurch gekennzeichnet, dass** der besagte mindestens eine Katalysator (18) auf einem flachen Vliesträger (20) angebracht wird.

4. Geschirrspüler/-trockner nach Anspruch 3, **dadurch gekennzeichnet, dass** ein Luftstrom (F) im Inneren des Belüftungskanals (11) tangential zum Träger (20) des besagten mindestens einen Katalysators (18) verläuft.

5. Geschirrspüler/-trockner nach einem beliebigen der Ansprüche 2 bis 4, **dadurch gekennzeichnet, dass** die Bestrahlung des besagten mindestens einen Katalysators (18) durch die besagte mindestens eine Ultraviolettstrahlenquelle (19) durch eine für Ultraviolettstrahlung durchlässige Wand des Belüftungskanals (11) erfolgt.

6. Geschirrspüler/-trockner nach einem beliebigen der Ansprüche 2 bis 5, **dadurch gekennzeichnet, dass** die besagte mindestens eine Ultraviolettstrahlenquelle (19) außerhalb des Belüftungskanals (11) angeordnet ist.

7. Geschirrspüler/-trockner nach einem beliebigen der Ansprüche 4 bis 6, **dadurch gekennzeichnet, dass** der Träger (20) des besagten mindestens einen Katalysators (18) in Abstand von einer unteren Wand (14) und einer oberen Wand (15) des Belüftungskanals (11) angeordnet ist.

8. Verfahren zur Geruchsbehandlung in einem Geschirrspüler/-trockner gemäß einem beliebigen der Ansprüche 1 bis 7, **dadurch gekennzeichnet, dass** die Vorrichtung zur Geruchsbehandlung (16) periodisch arbeitet.

9. Verfahren zur Geruchsbehandlung in einem Geschirrspüler/-trockner nach Anspruch 8, **dadurch gekennzeichnet, dass** die Vorrichtung zur Geruchsbehandlung (16) nach jedem Öffnen der Tür der Maschine (1) ausgelöst wird.

10. Verfahren zur Geruchsbehandlung in einem Geschirrspüler/-trockner nach Anspruch 8 oder 9, **dadurch gekennzeichnet, dass** die Vorrichtung zur Geruchsbehandlung (16) zyklisch außerhalb der Spülzyklen der besagten Maschine (1) ausgelöst wird.

11. Verfahren zur Geruchsbehandlung in einem Geschirrspüler/-trockner nach einem beliebigen der Ansprüche 8 bis 10, **dadurch gekennzeichnet, dass** die Vorrichtung zur Geruchsbehandlung (16) nach einem vorbestimmten Zeitraum, während welchem die Tür der besagten Maschine (1) nicht geöffnet wurde, auf Standby schaltet.

12. Verfahren zur Geruchsbehandlung in einem Geschirrspüler/-trockner nach einem beliebigen der Ansprüche 8 bis 11, **dadurch gekennzeichnet, dass** der Geschirrspüler/- trockner (1) mindestens eine Lufteinlassöffnung (8) in einer Wand (5) des Spültanks (2) aufweist, welche das Ansaugen der Gerüche durch mindestens einen Ventilator (12) ermöglicht, wobei die besagten Gerüche in einem Belüftungskanal (11) zirkuliert und die Vorrichtung zur Geruchsbehandlung (16) durch Photokatalyse durchlaufen, wobei die von der Vorrichtung zur Geruchsbehandlung (16) durch Photokatalyse behandelte Luft anschließend über mindestens eine Auslassöffnung (9) in einer Wand (10) des Spültanks (2) austritt.

13. Verfahren zur Geruchsbehandlung in einem Geschirrspüler/-trockner nach einem beliebigen der Ansprüche 8 bis 12, **dadurch gekennzeichnet, dass** die Dauer der Behandlung der Gerüche zwischen 4 und 20 Minuten pro Stunde beträgt.

14. Verfahren zur Geruchsbehandlung in einem Geschirrspüler/-trockner nach einem beliebigen der Ansprüche 12 bis 13, **dadurch gekennzeichnet, dass** der besagte mindestens eine Ventilator (12) und die besagte mindestens eine Ultraviolettstrahlenquelle (19) simultan für die Behandlung der Gerüche arbeiten.

15. Verfahren zur Geruchsbehandlung in einem Geschirrspüler/-trockner nach einem beliebigen der Ansprüche 8 bis 14, **dadurch gekennzeichnet, dass** die besagte mindestens eine Ultraviolettstrahlenquelle (19) während einer zusätzlichen Dauer über die Dauer der Behandlung der Gerüche hinaus arbeitet, um eine auf einem Träger (20), welcher den besagten mindestens einen Katalysator (18) umfasst, angebrachte Aktivkohleschicht zu regenerieren.

16. Verfahren zur Geruchsbehandlung in einem Geschirrspüler/-trockner nach Anspruch 15, **dadurch gekennzeichnet, dass** die Regeneration der besagten Aktivkohle (21) ohne Einschalten des mindestens einen Ventilators (12) erfolgt.
